# EUROPEAN PATENT SPECIFICATION

(11) **EP 2 833 932 B1**
(45) Date of publication and mention of the grant of the patent: **03.08.2022**
(21) Application number: 13716506.4
(22) Date of filing: 15.03.2013
(51) Int. Cl.: A61L 27/18, A61L 31/06, A61L 31/14, A61F 2/00, B29D 7/01, B29C 33/52, B29C 43/02, B29C 59/14, B81C 1/00, C08J 5/18, C08G 63/664, C08L 67/04, A61L 27/50, B29C 33/42, B29C 43/22, B29L 31/00, B29K 67/00, B29K 71/00

(54) **SUPER-HYDROPHILIC STRUCTURES**
HYDROPHILE STRUKTUREN
STRUCTURES SUPERHYDROPHILES

(30) Priority: 30.03.2012 US 201213435544
(43) Date of publication of application: 11.02.2015
(73) Proprietor: Ethicon Endo-Surgery, Inc., Cincinnati, OH 45242 (US); Agency for Science, Technology and Research, Singapore 138632 (SG)
(72) Inventor: LUONG-VAN, Emma, Kim, Dover Rise 138686 (SG); RODRIQUEZ, Isabel, Singapore 117602 (SG); LOW, Hong, Yee, Botannia 127723 (SG); ELMOUELHI, Noha, Randolph, New Jersey 07869 (US); COOPER, Kevin, Flemington, New Jersey 08822 (US); NATARAJAN, Sriram, Hillsborough, New Jersey 08844 (US); VYAKARNAM, Murty, N., Bridgewater, New Jersey 08807 (US); LIM, Chee Tiong, Sentosa Cove 098535 (SG)
(74) Representative: Carpmaels & Ransford LLP
(86) International application number: PCT/US2013/031822
(87) International publication number: WO 2013/148294

(56) References cited:
- EP-A2- 1 416 303
- WO-A1-2009/067482
- US-A1- 2012 052 234
- WAN Y ET AL: "Characterization of surface property of poly(lactide-co-glycolide) after oxygen plasma treatment", BIOMATERIALS, ELSEVIER SCIENCE PUBLISHERS BV., BARKING, GB, vol. 25, no. 19, 1 August 2004 (2004-08-01), pages 4777-4783, XP004505489, ISSN: 0142-9612, DOI: 10.1016/J.BIOMATERIALS.2003.11.051
- KEITH R. MILNER ET AL: "Submicron poly(L-lactic acid) pillars affect fibroblast adhesion and proliferation", JOURNAL OF BIOMEDICAL MATERIALS RESEARCH PART A, vol. 82A, no. 1, 1 July 2007 (2007-07-01), pages 80-91, XP055066847, ISSN: 1549-3296, DOI: 10.1002/jbm.a.31049
- JIANHUA WEI ET AL: "Influence of surface wettability on competitive protein adsorption and initial attachment of osteoblasts; Competitive protein adsorption and initial cell attachment", BIOMEDICAL MATERIALS, INSTITUTE OF PHYSICS PUBLISHING, BRISTOL, GB, vol. 4, no. 4, 1 August 2009 (2009-08-01), page 45002, XP020163011, ISSN: 1748-605X

## Description

This application is a continuation-in-part of, and claims the benefit of priority under 35 U.S.C. §120 to U.S. serial no. 12/871,745, filed August 30, 2010, and a continuation-in-part of U.S. serial no. 13/340,331, filed December 29, 2011.

### FIELD OF THE INVENTION

The present invention relates to polymer-based structures having shapes and mechanical properties that result in super-hydrophilicity.

### BACKGROUND OF THE INVENTION

Biomaterials are being used with increasing frequency for tissue substitution, such as for joint replacements and artificial organs. Tissue integration onto or into these biomaterials enhances long-term biocompatibility of these devices, and requires a form of eukaryocytic adhesion or compatibility with possible chemical integration to an implant surface. However, in what has been characterized as a "race for the surface", bacterial cells are known to compete with tissue cells for colonization onto the surfaces of such biomaterials. If bacterial cells are successful in colonizing an implant surface, devastating infections can occur which can require subsequent surgeries, or even result in sepsis and death, especially in immunosuppressed patients.

In "Biomaterial-Centered Infection: Microbial Adhesion Versus Tissue Integration", by Anthony G. Gristina, Science, Vol. 237, pp. 1588-1595 (1987), the author discusses the details of biological attraction and adhesion to various biomaterial implants and concludes that an optimum mechanism for preventing bacterial infection on such surfaces would be to develop biomaterial surfaces which encourage rapid eukaryocytic colonization of the surface, so as to impart the benefits of naturally derived antibacterial mechanisms produced by the living cells adhered to the surface.

There is an ongoing need for polymer-based structures having improved adsorption of biologics, e.g., proteins. Such structures can be suited for use in various applications, such as medical applications, e.g., medical diagnostics. It is especially desirable to provide structures whose surfaces have a specific, finely-tuned adsorption of biological materials.

U.S. Patent No. 5,246,451 discloses a vascular prosthesis made by coating a vascular graft material such as polyethylene terephthalate plasma coated with a fluoropolymer (PTFE) which is then treated with a plasma in a non-polymerizing gas atmosphere, e.g., oxygen, to improve biological entity binding to the fluoropolymer. The products of this disclosure rely on plasma treatment to improve protein binding and lack modified topography.

U.S. Patent No. 7,195,872 teaches providing substrates of high surface area with structural microfeatures that provide access to fluids and components therein. The substrates can be prepared by molding, embossing, photoresist techniques and can also be treated by etching, e.g., with argon, oxygen, helium, chlorine, SF₆, CF₄, and C₄F₈ gases. Surfaces can be modified by chemical treatments or radiative treatments, e.g., plasma treatment in gases. The reference emphasizes topography alone to bind proteins, or alternately, additional treatment with oxygen plasma to etch the surface and ammonia plasma for grafting amine groups on the surface.

"Characterization of surface property of poly(lactide-co-glycolide) after oxygen plasma treatment", Biomaterials, Vol. 25, pp. 4777-4783, 2004 concerns the characterization of surface property of poly(lactide-co-glycolide) after oxygen plasma treatment.

U.S. Patent Publication No. 2006/0154063 discloses a composite nanofiber construct comprising: at least a first nanofiber comprising at least a polymer and at least a calcium salt nanoparticle, wherein the ratio of polymer to calcium salt nanoparticle is between the range of 99:1 and 10:90 weight percent; and at least a second nanofiber comprising at least a polymer and at least a calcium salt nanoparticle, wherein the ratio of polymer to calcium salt nanoparticle is between the range of 100:0 and 70:30 weight percent. Hydrophilicity of the nanofibers is enhanced by plasma treatment, which lead to good adhesion and growing of cells.

U.S. Patent Publication No. 2008/0241512 discloses a chemical vapor deposition method of depositing layers of materials to provide super-hydrophilic surface properties, or super-hydrophobic surface properties, or combinations of such properties at various locations on a given surface. The invention also relates to electronic applications which make use of super-hydrophobic surface properties, and to biological applications which make use of super-hydrophilic surface properties.

U.S. Patent Publication No. 2012/0052234 A1 concerns an adhesive structure comprising a surface from which extend substantially cylindrical protrusions comprising a stiff resin having a Young's modulus of greater than 17 MPa.

It would be desirable to provide wettable polymer-based structures of substantially fixed topography having controllable adsorption of biologics, e.g., proteins, by adjusting characteristics of a substrate to provide a topography of enhanced surface area that is relatable or proportional to the surface adsorption of a biologic material.

### SUMMARY OF THE INVENTION

The present invention relates to a polydioxanone film comprising substantially cylindrical polydioxanone pillars on at least one side thereof, said pillars having average diameters from 1 µm to 3 µm, and heights of from 2 µm to 20 µm from the surface of the film, wherein the pillars are treated with an oxygen plasma to increase the oxygen content of said polydioxanone pillars, relative to untreated polydioxanone pillars.

In another embodiment, the invention is directed to a process for adsorbing proteins, comprising exposing a protein-containing solution to a polydioxanone film comprising substantially cylindrical polydioxanone pillars on at least one side thereof, said pillars having a diameter from 0.2 µm to 3 µm, and heights from 2 µm to 20 µm from the surface of the film.

In a further embodiment, the invention is directed to a medical device comprising a polydioxanone film comprising substantially cylindrical polydioxanone pillars on at least one side thereof, said pillars having a diameter from 1 µm to 3 µm, and heights of from 2 µm to 20 µm from the surface of the film, wherein the pillars are treated with an oxygen plasma to increase the oxygen content of said polydioxanone pillars, relative to untreated polydioxanone pillars.

### BRIEF DESCRIPTION OF THE DRAWINGS

FIG. 1 is a scanning electron microscope image of a super-hydrophilic PDO film having 3 micrometer diameter pillars extending from the surface of the film.
FIG. 2 is a scanning electron microscope image of a super-hydrophilic PDO film having 1 micrometer diameter pillars extending from the surface of the film.
FIG. 3 is a scanning electron microscope image of a super-hydrophilic PDO film having 0.6 micrometer diameter pillars extending from the surface of the film.
FIG. 4 is a graph comparing the protein (fibrinogen) adsorption capacities of the various embodiments of the invention, as compared to a flat film.

### DETAILED DESCRIPTION

The present invention is directed to high aspect ratio (HAR) polydioxanone (PDO) film having surface microstructures which demonstrates super-hydrophilic wetting characteristics, having an inherent static water drop contact angle of less than about 10°. It has been discovered that the super-hydrophilic nature of these structures enhances biologics, particularly protein adsorption onto the surfaces thereof.

The materials of the present invention are useful in various applications relying on biologic adsorption, e.g., protein adsorption, including diagnostic tests and other medical uses such as anastomosis devices, grafts, vascular prosthetic devices, soft tissue implants.

Biologics, for present purposes, include sugars, proteins, lipids, nucleic acids, polynucleotides or complex combinations of these substances, as well as living entities such as cells and tissues. Biologics can be isolated from a variety of natural sources-human, animal, or microorganism-and may be produced by biotechnology methods and other technologies. In some instances, biologics can be prepared using non-biological, chemical methods. Biologics include a wide range of medicinal products such as vaccines, blood and blood components, allergenics, somatic cells, gene therapy, tissues, and recombinant therapeutic proteins created by biological processes (as distinguished from chemistry).

Biologic adsorbent material made from polymer or comprising polymer can be formed into structures having high surface area topography. Structures can have tailored geometric features including substructures, e.g., pillars, with a diameter from 0.1-50 micrometers (100-50000 nm) and height greater than 1 micrometer (>1000 nm), which provide surface area greater than that of a substrate comprised of exposed flat surfaces. Protein adsorbency of a substrate is not dependent on surface area alone. It has now been found that in order to effectively utilize increased surface area of a substrate, adsorption by the substrate surface comprising substructures can be optimized to improve hydrophilicity/wettability. The polymeric structures according to the present invention of desirable high surface area topography exhibit improved biologics adsorption even without treating of the surfaces, e.g., by oxygen plasma.

In one aspect the present invention relates to a super-hydrophilic structure having a PDO film surface from which extend substantially cylindrical pillar-like protrusions, which protrusions are of sufficiently low diameter to promote adsorption of naturally occurring proteins.

When PDO pillars have average diameters from about 0.6 micrometer to about 1 micrometer, and heights of about 20 micrometers from the surface of the PDO film, the film has been found to demonstrate super-hydrophilicity, i.e. static water drop wetting angles of less than about 10°, in the absence of any post-treatment of the film/pillar surfaces. However, post-treatment by such as oxygen plasma treatment can be performed to enhance the hydrophilicity of larger diameter pillars, such as those having average diameters of about 3 micrometers.

In still another embodiment, the super-hydrophilic structure is integrally molded from a PDO resin or polymer, which is thermoplastic. By integrally molded is meant that the structure is formed in one piece, including its protrusions, from a mold. For present purposes, thermoplastic resin or polymer is a resin or polymer that softens when heated and hardens again when cooled.

In yet still another embodiment, the super-hydrophilic structure surface is substantially planar and the pillars are within ±45 degrees of normal to the planar surface, preferably within ±30 degrees of normal to the planar surface

In another embodiment, the super-hydrophilic structure has a pillar density of from 1 × 10⁵ to 6 × 10⁸ pillars/cm². For present purposes, "pillar density" can be described as the number of pillars present per square centimeter of super-hydrophilic structure surface. For example, the super-hydrophilic structure has a density of pillars on its surface ranging from about 1 × 10⁷ to about 5 × 10⁷ pillars per cm^{2.}

In yet another embodiment, the super-hydrophilic structure has a protein adsorption capacity from about 2.5 µg/cm² to about 6.0 µg/cm², or from about 4.0 µg/cm² to about 6.0 µg/cm² of the film surface.

The super-hydrophilic structure of the present invention can be at least partially formed by a process selected from nano- or micro-molding using a template, polymer self-assembly, lithography, and etching.

In another embodiment, the invention is directed to a process for adsorbing proteins, comprising exposing a protein-containing solution to a polydioxanone film comprising substantially cylindrical polydioxanone pillars on at least one side thereof, said pillars having diameters from 0.2 µm to 3 µm, and heights from 2 µm to 20 µm from the surface of the film. Optionally, the pillars are treated with an oxygen plasma to increase the oxygen content of the pillars, relative to untreated polydioxanone pillars.

In a further embodiment, the invention is directed to a medical device comprising a polydioxanone film comprising substantially cylindrical polydioxanone pillars on at least one side thereof, said pillars having diameters from 1 µm to 3 µm, and heights from 2 µm to 20 µm from the surface of the film. The medical devices can include medical implants, for example, anastomosis devices, grafts, vascular prosthetic devices and soft tissue implants.

The method for preparing a super-hydrophilic structure comprises: a) providing a specific solvent-dissolvable mold including pores or indentations; b) providing a meltable PDO polymer to the mold under conditions sufficient to permit filling the indentations of the mold by the polymer, said polymer being substantially non-dissolvable by the specific solvent; c) treating the mold and polymer of step b) to an extent sufficient to substantially solidify the polymer; and d) exposing the mold and polymer to the specific solvent under mold-dissolving conditions to provide a molded polymer substrate material comprising protrusions or pillars conforming to the indentations or pores of the mold. Optionally, this aspect further comprises at least one of the following conditions:
i) wherein the meltable polymer is provided to the mold as a softened film;
ii) wherein the mold comprises polycarbonate, the polymer is polydioxanone and the solvent is dichloromethane; and
iii) wherein step b) is carried out in a first stage and second stage, wherein the second stage is carried out at a greater pressure.

In one embodiment, the first stage is carried out at a temperature ranging from 90 to 110° C, pressure ranging from about 0 to about 20 kPa (about 0 to about 0.2 Bar), for a duration of 7 to 12 minutes, and the second stage is carried out at a temperature ranging from 90 to 110° C, pressure ranging from about 6 to about 20 kPa (about 0.06 to about 0.2 Bar), for a duration of 15 to 25 minutes.

In order to further enhance hydrophilicity, the PDO pillars and film surface can be treated with oxygen plasma, such as at 50 to 150 watts for 15 to 45 seconds, or at 75 to 125 watts for 25 to 35 seconds.

The invention is further explained in the description that follows with reference to the drawings illustrating, by way of non-limiting examples, various embodiments of the invention.

### Example 1

A 100 micrometers thick polydioxanone film with pillar-like structures on both sides was prepared. A polydioxanone film was compressed under heat and pressure between two 20 micrometers thick sheets of polycarbonate filter material. The filter material possesses microscopic (0.8 micrometer) holes. The polydioxanone film melted and flowed into the holes. After processing the sheet was annealed. The polycarbonate membrane filter was then dissolved in a bath of dichloromethane. The membrane filters used (0.8 micrometer ATTP, Cat No. ATTP14250, Lot No. R9SN70958 available from Millipore Corporation of Billerica, MA, USA) possessed two distinct sides. One side had a shiny appearance while the other was duller. A laminate for compression molding was constructed as follows:
a. A segment of polyimide film (sold under the tradename KAPTON by DuPont, Wilmington, DE) of 65-70 micrometers thickness was placed on a table;
b. A 15.2 cm (6 inch) polished square metal plate (thickness 0.8 mm) (shiny side up) was placed on the polyimide film;
c. A segment of polyimide film was placed on the 15.2 cm (6 inch) plate;
d. A 15.2 cm x 15.2 cm (6" x 6") x 80 micrometers steel shim with a 10.1 cm x 10.1 cm (4" x 4") cavity in the center was placed on the film;
e. A membrane filter was cut to fit in the shim cavity and was placed (dull side up) on the polyimide film;
f. A piece of 25 micrometers thick polydioxanone film was cut to fit in the shim cavity. The sample was placed on the membrane;
g. Another membrane filter (about 20 micrometers thick) was cut to fit in the shim cavity and placed (dull side down) on the polydioxanone film;
h. A segment of polyimide film was placed on the top membrane;
i. A 15.2 cm (6 inch) polished square metal plate (thickness 0.8 mm) (shiny side down) was placed on the polyimide film; and
j. Another segment of polyimide film was placed on the steel plate.

The resulting sample was loaded into a heated press with vacuum (less than 150 micrometers mercury) capability and was processed as follows:
a. The top and bottom platens were preheated to 220° C (428° F);
b. The sample was preheated under vacuum for 300 seconds prior to any compression;
c. The sample was compressed at 68948 kPa (10,000 psi) for 300 seconds;
d. The temperature was reduced to 21° C (70° F) while maintaining compression of 68948 kPa (10,000 psi);
e. The compressive force was released and the vacuum was purged; and
f. The sample was removed from the vacuum press.

The sample was annealed in the constrained condition (between two steel plates) in an inert environment (nitrogen gas) for a minimum of six hours at 70° C.

The polycarbonate membrane had been fused to the surface of the polydioxanone film. The membrane was removed from the surface of the polydioxanone film by immersing the sample in a bath of dichloromethane at room temperature for five minutes and was allowed to air dry prior to handling. Scanning electron microscope (SEM) images of the sample confirmed the presence of pillar-like structures which were about 20 micrometers high and 0.8 micrometer in diameter.

### Example 2

PDO pillars of 0.6, 1, and 3 micrometer diameters and heights of 20 micrometers were fabricated on PDO film essentially by the method set forth in Example 1, using commercial track etched polycarbonate membranes as a mold and a nanoimprinting process. Membranes having pores of 0.6 and 1 micrometer diameters and circular diameters of 4.5 cm, with thicknesses of 20 micrometers were obtained from Whatman, Florham Park, NJ. Membranes having pores of 3 micrometers diameter and circular diameters of 4.5 cm, with thicknesses of 20 micrometers were obtained from Millipore Corporation of Billerica, MA, USA. The membranes were used as templates to imprint a solvent-resistant PDO polymer film of 0.3 millimeter thickness, obtained from Ethicon, Inc. of Somerville, NJ, USA. The PDO film was pressed into the polycarbonate membrane templates under high temperature and pressures (120° C, 6000 kPa (60 bar)) for 5 minutes, melting the PDO. The PDO films with the imprinted PC membrane were placed between 2 metal plates and annealed for 3 hours at 70°C. The polymer structures were de-molded and released by dissolving the polycarbonate membrane in dicholoromethane. Films were immersed in two consecutive dichloromethane baths for 5 minutes per wash and then air dried.

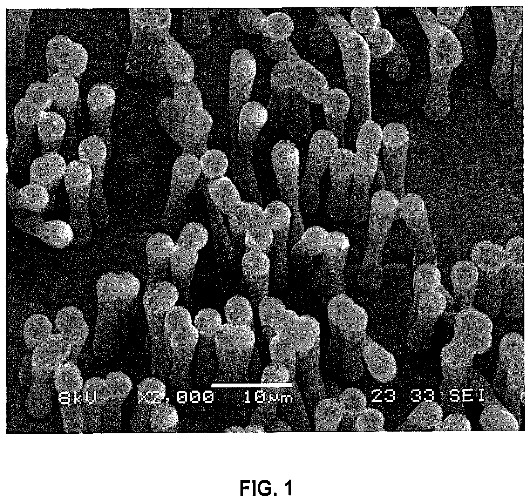

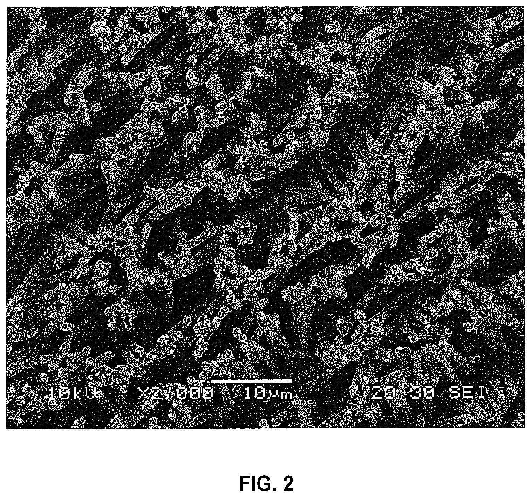

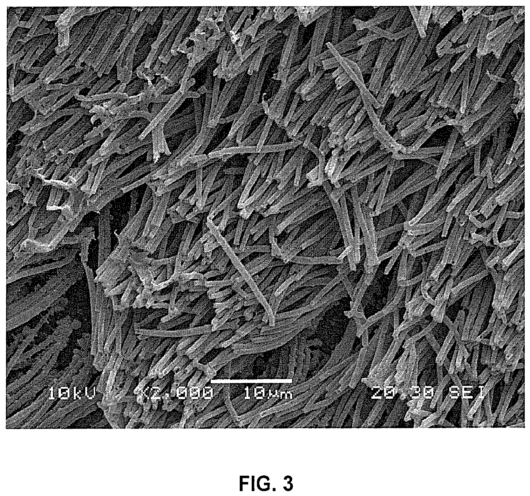

Scanning electron microscope (SEM) images of the samples confirmed the presence of pillar-like structures which were about 20 micrometers high. Figs. 1, 2, and 3, depict SEM images of the 3 µm diameter, 1 µm diameter, and 0.6 µm diameter HAR pillars, respectively.

The surface area ratio for these structures is more than 2 times the surface area of a flat film as shown in Table 1 below.

**TABLE 1**

| | **Flat film** | **3 µm HAR pillars** | **1 µm HAR pillars** | **0.6 µm HAR pillars** |
|---|---|---|---|---|
| **Surface Area Ratio** | 1 | 2 | 5 | 8 |

The surfaces of the PDO films were treated by oxygen plasma treatment using a microwave plasma processor (100 W, 30 seconds). Surface elemental analysis was conducted on the flat PDO films to determine the oxygen content of the film surfaces both before and after oxygen plasma treatment. The results are set forth in Table 2 below.

**TABLE 2**

| **Element** | **Untreated PDO** | **Plasma-Treated PDO** |
|---|---|---|
| C | 55.5% | 54.4% |
| O | 38.1% | 42.1% |

Static water contact angle measurements, herein referred to as contact angle measurements, were conducted using a sessile drop method. A Rame-Hart contact angle goniometer with Drop Image software was used. Plasma treatment was done immediately before contact angle measurement. Two microliter drops of de-ionized water were placed on the surface for measurement, and 5 measurements were taken for each surface. The mean contact angles are reported in Table 3.

**TABLE 3**

| | **Contact Angle Untreated** | **Contact Angle Plasma-Treated** |
|---|---|---|
| Flat film | 80° | 14° |
| 3 µm HAR pillars | 95° | spreads* |
| 1 µm HAR pillars | spreads | spreads |
| 0.6 µm HAR pillars | spreads | spreads |

| | | |
|---|---|---|
| *indicative of a contact angle of less than 10° | | |

Pillars with diameters 0.6 and 1 micrometer were wettable even without oxygen plasma treatment. The contact angle of PDO surfaces patterned with 3 µm pillars increased slightly compared to the flat film. Oxygen plasma treatment increased the wettability of the flat and 3 µm pillar surfaces.

The protein adsorption or protein uptake properties of these samples were evaluated by incubating the samples in protein solution and assaying using the bicinchoninic acid (BCA) assay. Films were cut into 1x1 cm pieces and incubated in 1 ml of fibrinogen protein solution (2mg/ml in phosphate buffered saline (PBS)) in a sealed 24-well plate overnight (18hrs) with orbital shaking. After protein incubation the films were removed from the wells and washed in 3 consecutive baths of PBS and then immediately quantified using the BCA Assay.

The BCA assay was conducted as follows: protein standards were made using the bovine serum albumin (BSA) standard provided in the BCA kit (QuantiPro BCA kit, Sigma Aldrich). Rinsed films were placed in wells of a 24-well plate containing 500 microliters PBS + 500 microliters BCA reagent (prepared as per kit instructions). For protein standards, 500 microliters of protein standard solution was placed in the well with 500 microliters BCA reagent. The plate was sealed and protected from light and incubated with orbital shaking at 50 rpm for 2 hrs at 37°C. After incubation, 200 microliters of the solution was transferred to wells of 96-well plate for absorbance reading at 562 nm.

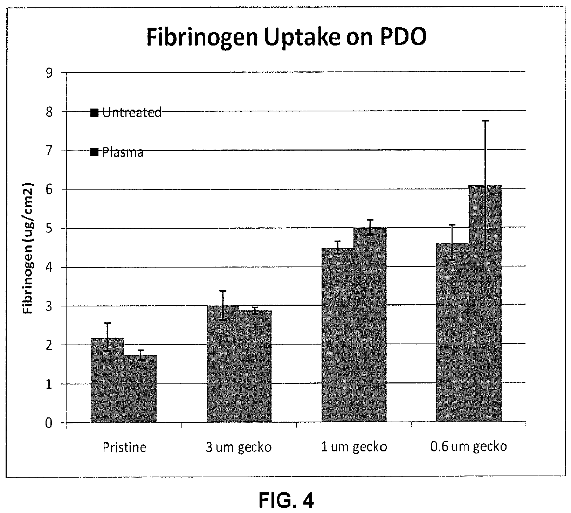

Increasing levels of fibrinogen adsorption were observed as the surface area of the sample increases. This trend was observed for both untreated and plasma-treated HAR pillar samples, since both exhibit similar water contact angle profiles. This trend was observed for fibrinogen as shown in FIG. 4.

When numerical lower limits and numerical upper limits are listed herein, ranges from any lower limit to any upper limit are contemplated.

## Claims

1. A polydioxanone film comprising substantially cylindrical polydioxanone pillars on at least one side thereof, said pillars having average diameters from 1 µm to 3 µm, and heights of from 2 µm to 20 µm from the surface of the film, wherein the pillars are treated with an oxygen plasma to increase the oxygen content of said polydioxanone pillars, relative to untreated polydioxanone pillars, and wherein the average diameters and the heights of the pillars are determined by scanning electron microscopy.

2. The polydioxanone film of claim 1, wherein the average diameter of the pillars is about 3 µm.

3. The polydioxanone film of claim 1, wherein the polydioxanone pillars have heights of about 20 µm from the surface of the film, and demonstrate super-hydrophilicity, having a static water contact angle of less than 10°, determined according to the sessile drop method indicated in the description.

4. The polydioxanone film of claim 1, which has a protein adsorption capacity from 2.5 µg/cm² to 6.0 µg/cm² of the film surface, evaluated by incubating the samples in protein solution and assaying using the bicinchoninic acid (BCA) assay indicated in the description.

5. The polydioxanone film of claim 4, which has a protein adsorption capacity from 4.0 µg/cm² to 6.0 µg/cm² of the film surface, evaluated by incubating the samples in protein solution and assaying using the bicinchoninic acid (BCA) assay indicated in the description.

6. A process for adsorbing proteins, comprising exposing a protein-containing solution to a polydioxanone film comprising substantially cylindrical polydioxanone pillars on at least one side thereof, said pillars having diameters from 0.2 µm to 3 µm, and heights from 2 µm to 20 µm from the surface of the is film, wherein the diameters and heights of the pillars are determined by scanning electron microscopy.

7. The process of claim 6, wherein the pillars are treated with an oxygen plasma to increase the oxygen content of said polydioxanone pillars, relative to untreated polydioxanone pillars.

8. The process of claim 6, wherein the polydioxanone pillars have diameters from 0.6 µm to 1 µm, heights of about 20 µm from the surface of the film, and demonstrate super-hydrophilicity, having a static water contact angle of less than 10°, determined according to the sessile drop method indicated in the description.

9. The process of claim 7, wherein the polydioxanone pillars have heights of about 20 µm from the surface of the film, and demonstrate super- hydrophilicity, having a static water contact angle of less than 10°, determined according to the sessile drop method indicated in the description.

10. The process of claim 6, wherein the polydioxanone film has a protein adsorption capacity from 2.5 µg/cm² to 6.0 µg/cm² of the film surface, evaluated by incubating the samples in protein solution and assaying using the bicinchoninic acid (BCA) assay indicated in the description.

11. The process of claim 10, wherein the polydioxanone film has a protein adsorption capacity from 4.0 µg/cm² to 6.0 µg/cm² of the film surface, evaluated by incubating the samples in protein solution and assaying using the bicinchoninic acid (BCA) assay indicated in the description.

12. A medical device comprising a polydioxanone film according to any of claims 1 to 5.

## Patentansprüche

1. Polydioxanon-Film, umfassend im Wesentlichen zylindrische Polydioxanon-Säulen auf wenigstens einer Seite davon, wobei die Säulen durchschnittliche Durchmesser von 1 µm bis 3 µm und Höhen von 2 µm bis 20 µm von der Oberfläche des Films aufweisen, wobei die Säulen mit einem Sauerstoffplasma behandelt werden, um den Sauerstoffgehalt der Polydioxanon-Säulen, im Verhältnis zu unbehandelten Polydioxanon-Säulen, zu erhöhen, und wobei die durchschnittlichen Durchmesser und die Höhen der Säulen durch Rasterelektronenmikroskopie bestimmt werden.

2. Polydioxanon-Film nach Anspruch 1, wobei der durchschnittliche Durchmesser der Säulen etwa 3 µm beträgt.

3. Polydioxanon-Film nach Anspruch 1, wobei die Polydioxanon-Säulen Höhen von etwa 20 µm von der Oberfläche des Films aufweisen und Superhydrophilie zeigen, mit einem statischen Wasserkontaktwinkel von weniger als 10°, bestimmt gemäß der in der Beschreibung angegebenen Sessile-Drop-Methode.

4. Polydioxanon-Film nach Anspruch 1, der eine Proteinadsorptionskapazität von 2,5 µg/cm² bis 6,0 µg/cm² auf der Filmoberfläche aufweist, bewertet durch Inkubation der Proben in Proteinlösung und Untersuchung mithilfe des in der Beschreibung angegebenen Bicinchoninsäure (BCA)-Assays.

5. Polydioxanon-Film nach Anspruch 4, der eine Proteinadsorptionskapazität von 4,0 µg/cm² bis 6,0 µg/cm² auf der Filmoberfläche aufweist, bewertet durch Inkubation der Proben in Proteinlösung und Untersuchung mithilfe des in der Beschreibung angegebenen Bicinchoninsäure (BCA)-Assays.

6. Verfahren zum Adsorbieren von Proteinen, umfassend Exponieren einer proteinhaltigen Lösung gegenüber einem Polydioxanon-Film, umfassend im Wesentlichen zylindrische Polydioxanon-Säulen auf wenigstens einer Seite davon, wobei die Säulen durchschnittliche Durchmesser von 0,2 µm bis 3 µm und Höhen von 2 µm bis 20 µm von der Oberfläche des Films aufweisen, wobei die Durchmesser und Höhen der Säulen durch Rasterelektronenmikroskopie bestimmt werden.

7. Verfahren nach Anspruch 6, wobei die Säulen mit einem Sauerstoffplasma behandelt werden, um den Sauerstoffgehalt der Polydioxanon-Säulen, im Verhältnis zu unbehandelten Polydioxanon-Säulen, zu erhöhen.

8. Verfahren nach Anspruch 6, wobei die Polydioxanon-Säulen Durchmesser von 0,6 µm bis 1 µm, Höhen von etwa 20 µm von der Oberfläche des Films aufweisen, und Superhydrophilie zeigen, mit einem statischen Wasserkontaktwinkel von weniger als 10°, bestimmt gemäß der in der Beschreibung angegebenen Sessile-Drop-Methode.

9. Verfahren nach Anspruch 7, wobei die Polydioxanon-Säulen Höhen von etwa 20 µm von der Oberfläche des Films aufweisen, und Superhydrophilie zeigen, mit einem statischen Wasserkontaktwinkel von weniger als 10°, bestimmt gemäß der in der Beschreibung angegebenen Sessile-Drop-Methode.

10. Verfahren nach Anspruch 6, wobei der Polydioxanon-Film eine Proteinadsorptionskapazität von 2,5 µg/cm² bis 6,0 µg/cm² auf der Filmoberfläche aufweist, bewertet durch Inkubation der Proben in Proteinlösung und Untersuchung mithilfe des in der Beschreibung angegebenen Bicinchoninsäure (BCA)-Assays.

11. Verfahren nach Anspruch 10, wobei der Polydioxanon-Film eine Proteinadsorptionskapazität von 4,0 µg/cm² bis 6,0 µg/cm² auf der Filmoberfläche aufweist, bewertet durch Inkubation der Proben in Proteinlösung und Untersuchung mithilfe des in der Beschreibung angegebenen Bicinchoninsäure (BCA)-Assays.

12. Medizinische Vorrichtung, umfassend einen Polydioxanon-Film nach einem der Ansprüche 1 bis 5.

## Revendications

1. Film de polydioxanone comprenant des piliers de polydioxanone sensiblement cylindriques sur au moins un côté de celui-ci, lesdits piliers possédant des diamètres moyens de 1 µm à 3 µm, et des hauteurs allant de 2 µm à 20 µm depuis la surface du film, les piliers étant traités avec un plasma d'oxygène pour augmenter la teneur en oxygènes desdits piliers de polydioxanone, par rapport aux piliers de polydioxanone non traités, et les diamètres moyens et les hauteurs des piliers étant déterminés par microscopie électronique à balayage.

2. Film de polydioxanone selon la revendication 1, le diamètre moyen des piliers étant d'environ 3 µm.

3. Film de polydioxanone selon la revendication 1, les piliers de polydioxanone possédant des hauteurs d'environ 20 µm depuis la surface du film et démontrant une superhydrophilie, possédant un angle statique de contact avec l'eau de moins de 10°, déterminé selon le procédé de la goutte sessile indiqué dans la description.

4. Film de polydioxanone selon la revendication 1, qui possède une capacité d'adsorption de protéines de 2,5 µg/cm² à 6,0 µg/cm² de la surface de film, évaluée par incubation des échantillons dans une solution de protéines et analyse en utilisant l'analyse à l'acide bicinchoninique (BCA) indiquée dans la description.

5. Film de polydioxanone selon la revendication 4, qui possède une capacité d'adsorption de protéines de 4,0 µg/cm² à 6,0 µg/cm² de la surface de film, évaluée par incubation des échantillons dans une solution de protéines et analyse en utilisant l'analyse à l'acide bicinchoninique (BCA) indiquée dans la description.

6. Procédé pour l'adsorption de protéines, comprenant l'exposition d'une solution contenant des protéines à un film de polydioxanone comprenant des piliers de polydioxanone sensiblement cylindriques sur au moins un côté de celui-ci, lesdits piliers possédant des diamètres de 0,2 µm à 3 µm, et des hauteurs allant de 2 µm à 20 µm depuis la surface du film, les diamètres et les hauteurs des piliers étant déterminés par microscopie électronique à balayage.

7. Procédé selon la revendication 6, les piliers étant traités avec un plasma d'oxygène pour augmenter la teneur en oxygènes desdits piliers de polydioxanone, par rapport aux piliers de polydioxanone non traités.

8. Procédé selon la revendication 6, les piliers de polydioxanone possédant des diamètres de 0,6 µm à 1 µm, des hauteurs d'environ 20 µm depuis la surface du film, et démontrant une superhydrophilie, possédant un angle statique de contact avec l'eau de moins de 10°, déterminé selon le procédé de la goutte sessile indiqué dans la description.

9. Procédé selon la revendication 7, les piliers de polydioxanone possédant des hauteurs d'environ 20 µm depuis la surface du film, et démontrant une superhydrophilie, possédant un angle statique de contact avec l'eau de moins de 10°, déterminé selon le procédé de la goutte sessile indiqué dans la description.

10. Procédé selon la revendication 6, le film de polydioxanone possédant une capacité d'adsorption de protéines de 2,5 µg/cm² à 6,0 µg/cm² de la surface de film, évaluée par incubation des échantillons dans une solution de protéines et analyse en utilisant l'analyse à l'acide bicinchoninique (BCA) indiquée dans la description.

11. Procédé selon la revendication 10, le film de polydioxanone possédant une capacité d'adsorption de protéines de 4,0 µg/cm² à 6,0 µg/cm² de la surface de film, évaluée par incubation des échantillons dans une solution de protéines et analyse en utilisant l'analyse à l'acide bicinchoninique (BCA) indiquée dans la description.

12. Dispositif médical comprenant un film de polydioxanone selon l'une quelconque des revendications 1 à 5.
